# EUROPEAN PATENT SPECIFICATION

(11) **EP 3 996 664 B1**
(45) Date of publication and mention of the grant of the patent: **21.08.2024**
(21) Application number: 20733470.7
(22) Date of filing: 23.06.2020
(51) Int. Cl.: A61K 8/34, A61K 8/44, A61Q 19/02

(54) **STABILIZATION OF RESORCINOL COMPOUNDS IN COSMETIC COMPOSITIONS**
STABILISIERUNG VON RESORCINOLVERBINDUNGEN IN KOSMETISCHEN ZUSAMMENSETZUNGEN
STABILISATION DE COMPOSÉS DE RÉSORCINOL DANS DES COMPOSITIONS COSMÉTIQUES

(30) Priority: 12.07.2019 EP 19186082
(43) Date of publication of application: 18.05.2022
(60) Divisional application: 24188885.8
(73) Proprietor: Unilever Global IP Limited, Wirral, Merseyside CH62 4ZD (GB); Unilever IP Holdings B.V., 3013 AL Rotterdam (NL)
(72) Inventor: ROSA, Jose, Guillermo, Trumbull, CT Connecticut 06611 (US); MOADDEL, Teanoosh, Trumbull, CT Connecticut 06611 (US)
(74) Representative: James, Helen Sarah
(86) International application number: PCT/EP2020/067468
(87) International publication number: WO 2021/008823

(56) References cited:
- EP-A1- 2 786 741
- WO-A1-2017/079905
- WO-A1-2018/114232
- US-B2- 6 863 897
- ALFRED HALPERN ET AL: "Stabilization of Resorcinol in Topical Preparations * *From the George A. Kelly, Sr. Pharmacy, Duquesne University, School of Pharmacy, Pittsburgh, Pa. Presented before the Section on Practical Pharmacy, AMERICAN PHARMACEUTICAL ASSOCIATION, Jacksonville, Fla., April, 1949.", JOURNAL OF THE AMERICAN PHARMACEUTICAL ASSOCIATION (PRACTICAL PHARMACY ED.), vol. 11, no. 1, 1 January 1950 (1950-01-01), US, pages 24 - 26, XP055650009, ISSN: 0095-9561, DOI: 10.1016/S0095-9561(16)32039-4

## Description

### Field of the invention

Provided herein are topical cosmetic compositions containing 4-substituted resorcinol derivatives which are stabilized with acetyl methionine

### Background of the invention

Beauty as seen by the beholder is influenced by numerous physiological and genetic factors. The basis for natural-looking appearance as beauty is in the skin, including the hair. Ideal skin should be smooth and even, with no apparent surface flaws in addition to having the transparent look with uniform color distribution. Skin and hair problems in aging individuals can result from a variety of extrinsic or intrinsic factors such as harmful UV radiation from the sun, exposure to the environment, stress, fatigue, disease, or a combination thereof.

Resorcinol (1,3-benzenediol) derivatives have been used to provide cosmetic benefits to skin and hair. 4-Substituted resorcinol derivatives and their preparation have been used for skin lightening and other cosmetic uses; see, for example, U.S. Patent No. 4,959,393, U.S. Patent No. 6,132,740, U.S. Patent Application Publication No. 2008/0131382, and Japanese Published Patent Application Nos. JP 2001-01 0925 and JP 2000-327557. Resorcinol-type skin lightening agents, which can be synthesized using coumarin as starting material, are disclosed in U.S. Patent Application Publication No. 2004/0042983. However, some of these compounds can be difficult to formulate, as they are unstable in a cosmetically acceptable vehicle.

Oxidative instability is an undesirable characteristic in 4-substituted resorcinols. There is a need, therefore, for an agent that will stabilize 4-substituted resorcinols against oxidation. In particular, there is a need for an agent that will prevent the oxidation of 4-substituted resorcinols.

It would be desirable to have topical cosmetic compositions that contain resorcinol derivatives that are stabilized against factors such as discoloration, oxidation, general storage stability. The compositions comprising 4-alkyl resorcinols stabilized with N-acetyl methionine (AceMet) described herein fill this need.

### BRIEF DESCRIPTION

The present invention is disclosed in the appended claims.

The present disclosure relates to a stabilized topical cosmetic composition comprising:
a. 0.00001 to 10 wt.% of said composition of N-Acetyl Methionine;
b. 0.00001 to 10 wt.% of said composition of one or more 4-substituted resorcinol derivatives of the general formula (1) and
c. a cosmetically acceptable vehicle.

The present disclosure further relates to a method of stabilizing 4-ethyl resorcinol or 4-hexyl resorcinol in a composition comprising a cosmetically acceptable vehicle by adding to the composition Acetyl Methionine in an amount sufficient to stabilize said 4-ethyl resorcinol or 4-hexyl resorcinol.

Another aspect which is disclosed in the present disclosure is the use of N-Acetyl Methionine for stabilizing 4-ethyl resorcinol or 4-hexyl resorcinol in a composition comprising a cosmetically acceptable vehicle

The 4-substituted resorcinol derivative is stabilized against degradation/discoloration/oxidation with inclusion of N-acetyl methionine in the composition. The compositions comprise cosmetically effective amounts of the resorcinol compounds and N-acetyl methionine in an amount sufficient to stabilize the resorcinol compounds against chemical degradation as evidenced by discoloration. The resorcinol derivatives provided herein, which are defined below, are useful in the treatment or prevention of one or more cosmetic conditions as desired by the subject in need thereof, such as to prevent, lighten, reduce or treat the signs or appearance of undesired pigmentation of skin affected by the one or more conditions.

The 4-substituted resorcinol derivatives used in the stabilized compositions have the general formula I:
**R₂O** 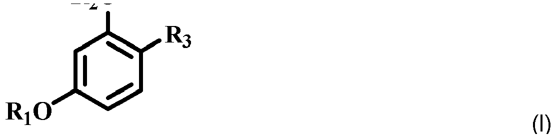
wherein each R₁ and R₂ independently is selected from the group consisting of a hydrogen atom, -CO-R, -COO-R, and CONHR; wherein R represents a C₁ - C₁₈ hydrocarbon;
R₃ represents a C₁ - C₁₈ hydrocarbon or has a general formula (II): 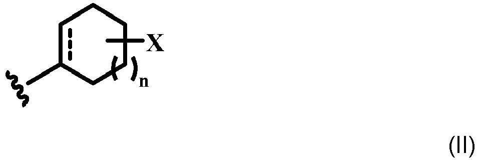
wherein X represents hydrogen; OR¹, wherein R¹ represents hydrogen, (C₁ - C₆)alkyl or aryl-(C₁-C₆)alkyl; OCOR² wherein R² represents (C₁-C₆)alkyl, aryl-(C₁-C₆)alkyl or phenyl; (C₁-C₆)alkyl; aryl-(C₁-C₆)alkyl; or NHR¹ wherein R¹ is defined as above;
n is 0 to 3; and
the dashed line indicates an optional double bond;
and preferably include 4-linear alkyl resorcinols, 4-branched alkyl resorcinols, 4-cycloalkyl resorcinols, and mixtures thereof.

More particular preferred resorcinol derivatives include 4-ethyl resorcinol, 4-isopropyl resorcinol, 4-hexyl resorcinol and 4-cyclohexyl resorcinol, as well as O-acylated versions thereof.

In another embodiment, provided is a stabilized cosmetic composition comprising:
a. a 4-substituted resorcinol derivative stabilized by N-acetyl methionine;
b. an optional skin benefit agent; and
c. a cosmetically acceptable vehicle;

wherein said is N-acetyl methionine present in an amount of about 0.0001 wt % to about 5 wt % of said cosmetic composition; and
wherein the weight ratio of said N-acetyl methionine to said 4-substituted resorcinol derivative is about 10,000:1 to about 1:100,000.

The compositions are aesthetically pleasing and the resorcinol derivatives therein have improved storage/color/oxidative stability.

Also provided is a method of stabilizing (against oxidation, discoloration, storage, etc) 4-hexyl resorcinol in a composition comprising a cosmetically acceptable vehicle by adding to the composition N-acetyl methionine in an amount sufficient to stabilize said 4-hexyl resorcinol.

Also provided is the use of the N-acetyl methionine and 4-substituted resorcinol derivatives of any in the manufacture of a cosmetic composition for topical application to the skin of an individual in need thereof.

In another variation, provided herein is a method of regulating a skin condition by topically applying an effective amount of the inventive compositions comprising 4-substituted resorcinol derivatives and N-acetyl methionine to the skin of an individual in need thereof

Also provided herein is a product comprising instructions directing a user to apply a composition including a skin care composition comprising one or more 4-substituted resorcinol derivatives, which compositions also contain N-acetyl methionine as a stabilizer. The composition can comprise pharmaceutically and/or dermatologically acceptable carriers and vehicles.

The compositions are aesthetically pleasing and have improved storage/oxidative stability.

### Detailed description of the invention

### Definitions

As used herein, a "individual" is a mammal, particularly a human. It is understood that use "in" an individual can comprise use "on" an individual as well; that is, use "in" an individual can comprise either internal use, external use, or both, according to the context of the use.

As used herein, "administering to skin in need of such treatment" means contacting (e.g., by use of the hands or an applicator such as, but not limited to, a wipe, tube, roller, spray, or patch) the area of skin in need such treatment or an area of skin proximate to the area of skin in need of such treatment.

As used herein, "composition" means a composition suitable for topical administration to the skin.

As used herein, the term "cosmetic composition" is intended to describe compositions for topical application to human skin, including leave-on and wash-off products.

The term "skin" as used herein includes the skin on the face, neck, chest, back, arms, axillae, hands, legs, and scalp of a human.

Except in the examples, or where otherwise explicitly indicated, all numbers in this description indicating amounts of material or conditions of reaction, physical properties of materials and/or use are to be understood as modified by the word "about". All amounts are by weight of the composition, unless otherwise specified.

For the avoidance of doubt the word "comprising" is intended to mean including but not necessarily consisting of or composed of. In other words the listed steps or options need not be exhaustive.

For all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the skin or the skin condition being treated, but do not contain any other components which substantially affect the skin or the skin condition being treated other than those components expressly listed; or, if the composition does contain extra components other than those listed which substantially affect the skin or the skin condition being treated, the composition does not contain a sufficient concentration or amount of the extra components to substantially affect the skin or the skin condition being treated. When a method is described as "consisting essentially of" the listed steps, the method contains the steps listed, and may contain other steps that do not substantially affect the skin or the skin condition being treated, but the method does not contain any other steps which substantially affect the skin or the skin condition being treated other than those steps expressly listed.

As used herein, the term "cosmetically-acceptable" or "dermatologically-acceptable" means that the compositions or components thereof so-described are suitable for use in contact with skin, particularly human skin, without undue toxicity, incompatibility, instability, irritation, or allergic response.

As used herein, the term "cosmetically acceptable carrier", "cosmetically acceptable excipient", "dermatologically acceptable carrier" or "dermatologically acceptable excipient" includes all solvents, dispersion media, coatings, antibacterial and antifungal agents, isotonic and absorption delaying agents and the like, that are cosmetically acceptable or dermatologically acceptable. Except insofar as any conventional media or agent is incompatible with the active ingredient, its use in the cosmetic compositions is contemplated. Supplementary active ingredients can also be incorporated into the compositions. Cosmetically acceptable carriers are suitable for topical application to the skin, have good aesthetic properties, are compatible with the active agents described herein and any other components, and cause minimal or no safety or toxicity concerns. A safe and effective amount of carrier is from about 50% to about 99.99% or about 50% to about 99%, preferably from about 80% to about 99.9% or about 75% to about 99%, more preferably from about 90% to about 98%, and most preferably from about 90% to about 95% or about 85% to about 95% of the composition. The percentages are preferably percent by weight.

As used herein, the terms "skin condition", "dermatologic condition", and "dermatological condition" are used interchangeably.

As used herein, the term "sunscreen" may include, but is not limited to, organic or inorganic sunscreens, such as methoxycinnamate, oxybenzone, avobenzone, and the like; sun blocks such as titanium oxide and zinc oxide; and skin protectants; or mixtures thereof.

As used herein, the term "topical application" means to apply or spread the compositions described herein onto the surface of the skin.

As used herein, "alkyl" is intended to embrace a saturated linear or branched hydrocarbon chain having the number of carbon atoms specified. In one embodiment, alkyl groups can have 1 to 12 carbon atoms. "Alkylene" is intended to embrace a divalent saturated linear or branched hydrocarbon chain having the number of carbon atoms specified. In one embodiment, alkylene groups can have 1 to 12 carbon atoms.

As used herein, "cycloalkyl" is intended to embrace a saturated cyclic hydrocarbon chain having the number of carbon atoms specified. In one embodiment, cycloalkyl groups can have 3 to 12 carbon atoms.

As used herein, "alkenyl" is intended to embrace a linear or branched hydrocarbon chain having at least one carbon-carbon double bond. In one embodiment, alkenyl groups can have 2 to 12 carbon atoms.

As used herein, "aryl" is intended to embrace an aromatic hydrocarbon such as for example a substituted or unsubstituted phenyl or naphthyl group.

While the compounds described herein can occur and can be used as the neutral (non-salt) compound, the description is intended to embrace all salts of the compounds described herein, as well as methods of using such salts of the compounds. In one embodiment, the salts of the compounds comprise pharmaceutically acceptable salts and/or dermatologically acceptable salts. Cosmetically acceptable salts are those salts which can be applied to the skin of humans and/or animals and which, upon application, retain at least some of the biological activity of the free compound (neutral compound or non-salt compound). The desired salt of a basic compound may be prepared by methods known to those of skill in the art by treating the compound with an acid.

Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N-dibenzylethylenediamine, triethylamine or trialkylamine salts. Salts of acidic compounds with amino acids, such as lysine salts, can also be prepared.

### Compositions

### 4-Substituted Resorcinol Derivatives

Resorcinol derivatives are known compounds and can be readily obtained, for example, by a method wherein a saturated carboxylic acid and resorcinol are condensed in the presence of zinc chloride and the resultant condensate is reduced with zinc amalgam/hydrochloric acid (Lille. J. Bitter, LA. Peiner. V, Tr. Nauch-Issled. Inst. slantsev 1969, No. 18, 127), or by a method wherein resorcinol and a corresponding alkyl alcohol are reacted in the presence of an alumina catalyst at a high temperature of from 200 to 400° C (British Patent No. 1 ,581 ,428).

The compositions comprise as an effective amount of a resorcinol derivative of the following formula (I):

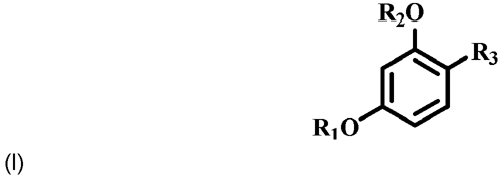

Each R₁ and R₂, independently, represents a hydrogen atom, -CO-R (acyl group), -COO-R, CONHR; the latter three represented by the following formula A, respectively: 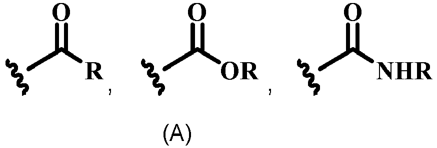 where R represents saturated or unsaturated, linear branched or cyclic C₁ - C₁₈ hydrocarbon. In a preferred embodiment, each or both R₁ and/or R₂ represents hydrogen. In a more preferred embodiment, both R₁ and R₂ represent hydrogen.

### R₃ represents:

(1) saturated or unsaturated, linear branched or cyclic C₁ - C₁₈ hydrocarbon, preferably a C₂ - C₁₂ hydrocarbon;
   or
(2) a group of general formula (II) 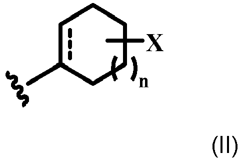
   Wherein X is hydrogen; OR¹, wherein R¹ represents hydrogen, (C₁ - C₆)alkyl or aryl-(C₁-C₆)alkyl; OCOR² wherein R² represents (C₁-C₆)alkyl, aryl-(C₁-C₆)alkyl or phenyl; (C₁-C₆)alkyl; aryl-(C₁-C₆)alkyl; or NHR¹ wherein R¹ is defined as above;
   wherein n is 0 to 3; and wherein the dashed line indicates an optional double bond.

For example, where n is 0, the group of general formula II is a 5-membered ring; where n is 1, the group is a 6-membered ring; where n is 2, a 7-membered ring; and where n is 3, an 8-membered ring.

### 4- Substituted Resorcinols: Linear or Branched

In the above formula (1), the group represented by R₃ and preferably having from 2 to 12 carbon atoms, where the arrangement is linear, may include an ethyl group, a propyl group, a butyl group, a pentyl group, a hexyl group, a heptyl group, an octyl group, a nonyl group, a decyl group, an undecyl group and a dodecyl group. These linear alkyl groups may be substituted with a methyl or ethyl group at one or more hydrogen atoms thereof. Specific examples of the substituted alkyl group include an isopropyl group, an isobutyl group, an isoamyl group, a 2-methylhexyl group and the like. Preferred alkyl groups are those where R₃ is an ethyl, propyl, isopropyl, butyl, pentyl, hexyl, heptyl, or octyl group. The most preferable alkyl resorcinols are those where R₃ is an ethyl, isopropyl, or hexyl group.

### 4-Cycloalkyl Resorcinols

In the case resorcinol derivatives of formula (I) where R₃ represents a structure of the general formula (II) shown hereinabove, the resorcinol derivatives are referred to herein as 4-cycloalkyl resorcinols, and are represented by the general formula (III):

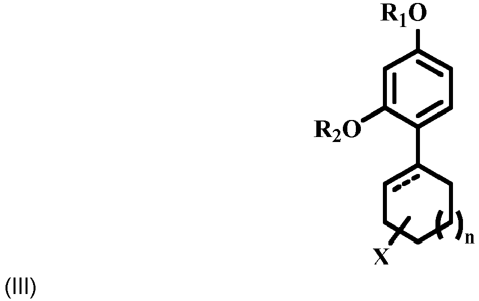

In the general formula (III) (as well as formula (II) hereinabove):
X represents hydrogen; OR¹, wherein R¹ represents hydrogen, (C₁ - C₆)alkyl or aryl-(C₁-C₆)alkyl; OCOR² wherein R² represents (C₁-C₆)alkyl, aryl-(C₁-C₆)alkyl or phenyl; (C₁-C₆)alkyl; aryl-(C₁-C₆)alkyl; or NHR¹ wherein R¹ is defined as above;
n is 0 to 3; and the dashed line indicates an optional double bond at that position.

Examples of more specific embodiments of the 4-cycloalkyl resorcinols include:
(a) compounds of the formula (III) wherein a single bond connects the two carbon atoms at the dashed line;
(b) compounds of the formula (III) wherein n is one;
(c) compounds of the formula (III) wherein X is hydrogen;
(d) compounds of the formula (III) wherein X is hydrogen, methyl or ethyl;
(e) compounds of the formula (III) wherein n is zero;
(f) compounds of the formula (III) wherein n is two; and
(g) compounds of the formula (III) wherein X is benzyloxy.

Preferred compounds of formula (III) are 4-cyclohexyl resorcinol, 4-cycloheptyl resorcinol, and 4-cyclooctyl resorcinol. Most preferred compound of formula (III) is 4-cyclohexyl resorcinol.

The amount of the resorcinol derivative is in the range of 0.00001 % to 10 %, preferably 0.001 to 7 %, most preferably 0.01 to 5 %, by weight of the total amount of a cosmetic composition.

### N-Acetyl Amino Acids

We have now found that N-Acetyl Amino Acids inhibit the oxidation of 4-substituted resorcinol derivatives. Preferably, in accordance with the present invention, N-Acetyl Amino Acids are used to stabilize 4-substituted resorcinols incorporated in cosmetic compositions.

Acetyl methionine is the N-acetyl derivative of the amino acid methionine. N-AcetylMethionine ("AceMet") is preferred and, for purposes of the present invention, has the following chemical structure:

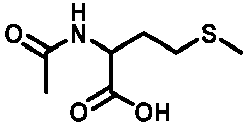

Acetyl methionine (CAS No 1115-47-5 for Acetyl-DL-methionine) and CAS No 65-82-7 for N-Acetyl-L-methionine ("AceMet") have been included in cosmetic applications. For purposes of the present invention, N-Acetyl-L-methionine ("AceMet") is preferred, due to its enhanced solubility profile (water solubility, logP and logO) over L-methionine.

AceMet is a nutritionally and metabolically equivalent form of the essential amino acid L-methionine (L-Met), the latter used as an intermediate for the biosynthesis of S-Adenosyl methionine (SAMe) - an essential nutrient for maintaining an optimal cellular energy balance - as well as many important biomolecules including cysteine, carnitine, taurine, lecithin, phosphatidylcholine. AceMet has also been shown to have detoxifying properties (e.g. antidote for paracetamol overdose, detoxification of benzene derivatives), and is presumed stable in formulation (WO0040217, WO05055947). N-acetylamino acids (e.g. AceMet) have been used as part of a combination of multiple actives for topical cosmetic applications to treat skin disorders (associated with aging, hair, nails, etc.) (WO 00/40717 to Tristrata Inc).

N-acetyl methionine is available from multiple suppliers including Sigma-Aldrich.

The inventive compositions contain 0.00001 to 10% by weight of the total amount of said compositions of AceMet and about 0.00001 to about 50 % of 4-substituted resorcinols. The particular advantage of the inventive compositions is that 4-substituted resorcinols can be stabilized by AceMet against oxidation.

An amount of AceMet effective to inhibit the oxidation of 4-substituted resorcinol derivative may be determined by experimentation. The AceMet and 4-substituted resorcinol derivatives are present in the composition in a weight ratio of 1:10000 to 10000:1 of AceMet:resorcinol, preferably 1:1000 to 1:5000, more preferably 1:1 to 1:1000.

Preferably, the amount of AceMet in the cosmetic composition is in the range of 0.00001 % to 10 %, more preferably 0.0001 % to 5 wt %, most preferably 0.0001 % to 2% by weight of the total amount of said composition.

### OPTIONAL COMPONENTS

Any of the compounds described herein can be mixed with other components. Examples of such components include oily components such as hydrocarbons, fats and oils such as liquid paraffin, squalene, petroleum jelly such as Vaseline^{®} (a registered trademark of Conopco Corp., Englewood Cliffs, New Jersey), cetyl alcohol, isostearyl alcohol, cetyl-2-ethylhexanoate, 2-octyldodecyl alcohol, glycerin, glycerin triisostearate, nut oils, and lanolin, as well as wax, silicone, surfactants, thickeners, neutralizers, antiseptics, germicides, anti-oxidants, powder components, pigments, perfumes, ultraviolet light absorbents, drugs, metallic sealant, and pH modifiers.

### SKIN BENEFIT AGENTS

The cosmetic compositions of the present invention may be used in personal care and personal wash products, such as, for example, lotions, soaps, deodorants, etc. The N-acetyl methionine compounds and the stabilizing agents that form the compositions of the present invention may have other useful functions when incorporated in personal care compositions.

Preferred personal care compositions are those suitable for the application to human skin, which optionally, but preferably, include a skin benefit agent. Suitable skin benefit agents include anti-aging, wrinkle-reducing, skin whitening, anti-acne, and sebum reduction agents. Examples of these include alpha-hydroxy acids and esters, beta-hydroxy acids and esters, polyhydroxy acids and esters, kojic acid and esters, ferulic acid and ferulate derivatives, vanillic acid and esters, dioic acids (such as sebacid and azoleic acids) and esters, retinol, retinal, retinyl esters, hydroquinone, t-butyl hydroquinone, mulberry extract, licorice extract, 1-methylnicotinamide chloride and resorcinol derivatives other than the 4-substituted resorcinol derivatives discussed hereinabove.

The active compounds described herein can also be used in combination with skin peeling agents (including glycolic acid or trichloroacetic acid face peels) or skin exfoliating agents (including retinoids, such as retinoic acid or retinol) to lighten skin tone and prevent repigmentation. The appropriate dose regimen, the amount of each dose administered, and specific intervals between doses of the active compound will depend upon the particular active compound employed, the condition of the patient or subject being treated, and the nature and severity of the disorder or condition being treated. Preferably, the active compound is administered in an amount and at an interval that results in the desired treatment of or improvement in the disorder or condition being treated.

An active compound described herein can also be used in combination with sun screens (UVA or UVB blockers, absorbers, diffusing agents) to prevent or protect against sun or UV-induced skin damage.

In some variations, the composition further comprises ascorbic acid, its derivatives and ascorbic-acid based products (such as magnesium ascorbate) or other products with an anti-oxidant mechanism (such as resveratrol, tocopherols, tocotrienols and derivatives).

In some variations, the composition further comprises a soybean extract that is a blend of compounds isolated from soybean. The soybean extract may contain only a portion of the soybean (e.g., an extract of the soybean such as a lipid reduced soybean powder or filtered soymilk) or may contain the entire soybean (e.g., a ground powder of the soybean). The soybean extract may be in the form of a fluid (e.g., soymilk) or a solid (e.g., a soybean powder or soymilk powder).

The appropriate dose regimen, the amount of each dose administered, and specific intervals between doses of each active topical skin benefit agent will depend upon the specific combination of active agents employed, the condition of the subject in need thereof, and the nature and severity of the subject's topical cosmetic condition.

In the cosmetic compositions provided herein, the concentration of the active compound is generally between 0.01% and 10%, or between about 0.01% and about 10%, for example between 0.1% and 5% or between about 0.1% and about 5%, or between 0.1% and 2%, or between about 0.1% and about 2%, or between 0.1% and 1%, or between about 0.1% and about 1%, relative to the total weight of the composition.

The compositions described herein can be applied directly to the skin. Alternatively, they can be delivered by various transdermal delivery systems, such as transdermal patches as known in the art. For example, for topical administration, the active ingredient can be formulated in a solution, gel, lotion, ointment, cream, suspension, paste, liniment, powder, tincture, aerosol, patch, or the like in a cosmetically acceptable form by methods known in the art. The composition can be any of a variety of forms common in the cosmetic arts for topical application to animals or humans, including solutions, lotions, sprays, creams, ointments, salves, gels, etc., as described below. Exemplary agents are those that are viscous enough to remain on the treated area, those that do not readily evaporate, and/or those that are easily removed by rinsing with water, optionally with the aid of soaps, cleansers and/or shampoos.

The compositions may be made into a wide variety of product types that include but are not limited to solutions, suspensions, lotions, creams, gels, toners, sticks, sprays, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, make-up such as foundations, eye liners, and eye shadows, and the like. These product types may contain several types of cosmetically-acceptable carriers including, but not limited to solutions, suspensions, emulsions such as microemulsions and nano-emulsions, gels, solids and liposomes.

### COSMETICALLY ACCEPTABLE VEHICLE

The cosmetically acceptable vehicle may act as a dilutant, dispersant or carrier for the skin benefit ingredients in the composition, so as to facilitate their distribution when the composition is applied to the skin.

The vehicle may be aqueous, anhydrous or an emulsion. Preferably, the compositions are aqueous or an emulsion, especially water-in-oil or oil-in-water emulsion, preferentially oil in water emulsion. Water when present will be in amounts which may range from 5 to 99%, preferably from 20 to 70%, optimally between 40 and 70% by weight.

Besides water, relatively volatile solvents may also serve as carriers within compositions of the present invention. Most preferred are monohydric C₁-C₃ alkanols. These include ethyl alcohol, methyl alcohol and isopropyl alcohol. The amount of monohydric alkanol may range from 1 to 70%, preferably from 10 to 50%, optimally between 15 to 40% by weight.

Emollient materials may also serve as cosmetically acceptable carriers. These may be in the form of silicone oils and synthetic esters. Amounts of the emollients may range anywhere from 0.1 to 50%, preferably between 1 and 20% by weight.

Silicone oils may be divided into the volatile and non-volatile variety. The term "volatile" as used herein refers to those materials which have a measurable vapor pressure at ambient temperature. Volatile silicone oils are preferably chosen from cyclic or linear polydimethylsiloxanes containing from 3 to 9, preferably from 4 to 5, silicon atoms. Linear volatile silicone materials generally have viscosities less than about 5 centistokes at 25°C while cyclic materials typically have viscosities of less than about 10 centistokes. Nonvolatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about 5 to about 25 million centistokes at 25°C. Among the preferred non-volatile emollients useful in the present compositions are the polydimethyl siloxanes having viscosities from about 10 to about 400 centistokes at 25°C.

Among the ester emollients are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
(4) Wax esters such as beeswax, spermaceti, myristyl myristate, stearyl stearate and arachidyl behenate.
(5) Sterols esters, of which cholesterol fatty acid esters are examples.

Fatty acids having from 10 to 30 carbon atoms may also be included as cosmetically acceptable carriers for compositions of this invention. Illustrative of this category are pelargonic, lauric, myristic, palmitic, stearic, isostearic, hydroxystearic, oleic, linoleic, ricinoleic, arachidic, behenic and erucic acids.

Humectants of the polyhydric alcohol-type may also be employed as cosmetically acceptable carriers in compositions of this invention. The humectant aids in increasing the effectiveness of the emollient, reduces skin dryness and improves skin feel. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1 ,2,6-hexanetriol, ethoxylated glycerol, propoxylated glycerol and mixtures thereof. The amount of humectant may range anywhere from 0.5 to 30%, preferably between 1 and 15% by weight of the composition.

Thickeners may also be utilized as part of the cosmetically acceptable carrier of compositions according to the present invention. Typical thickeners include crosslinked acrylates (e.g. Carbopol 982), hydrophobically-modified acrylates (e.g. Carbopol 1382), cellulosic derivatives and natural gums. Among useful cellulosic derivatives are sodium carboxymethylcellulose, hydroxypropyl methylcellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Amounts of the thickener may range from 0.0001 to 5%, usually from 0.001 to 1%, optimally from 0.01 to 0.5% by weight.

Collectively the water, solvents, silicones, esters, fatty acids, humectants and/or thickeners will constitute the cosmetically acceptable carrier in amounts from 1 to 99.9%, preferably from 80 to 99% by weight.

An oil or oily material may be present, together with an emulsifier to provide either a water-in-oil emulsion or an oil-in-water emulsion, depending largely on the average hydrophilic-lipophilic balance (HLB) of the emulsifier employed.

Surfactants may also be present in cosmetic compositions of the present invention. For leave-on products, total concentration of the surfactant will range from 0.1 to 40%, preferably from 1 to 20%, optimally from 1 to 5% by weight of the composition. For wash-off products, such as cleansers and soap, total concentration of surfactant will range at about 1 to about 90 %. The surfactant may be selected from the group consisting of anionic, nonionic, cationic and amphoteric actives. Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, acyl glutamates, C₈-C₂₀ alkyl ether phosphates and combinations thereof.

The inventive cosmetic compositions optionally contain a lathering surfactant. By a "lathering surfactant" is meant a surfactant which, when combined with water and mechanically agitated, generates a foam or lather. Preferably, the lathering surfactant should be mild, meaning that it must provide sufficient cleansing or detergent benefits but not overly dry the skin, and yet meet the lathering criteria described above. The cosmetic compositions of the present invention may contain a lathering surfactant in a concentration of about 0.01 % to about 50 %.

The compositions can be formulated as solutions. Solutions typically include an aqueous or organic solvent, e.g., from about 50% to about 99.99% or from about 90% to about 99% of a cosmetically acceptable aqueous or organic solvent. Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, and mixtures thereof. One example of such solvents is a mixture of ethanol/polyethylene glycol (80/20).

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water. Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook (International Cosmetic Ingredient Dictionary and Handbook) pp. 1693-1697.

The compositions described herein can also be formulated as emulsions. If the carrier is an emulsion, from about 1% to about 10% (e.g., from about 2% to about 5%) of the carrier contains an emulsifier(s). Emulsifiers may be nonionic, anionic or cationic. Examples of emulsifiers include, but are not limited to, those set forth in the ICI Handbook, pp. 1673-1686.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the art and are useful in compositions and methods described herein. Multiphase emulsion compositions, such as the water-in-oil-in-water type or the oil-in-water-in-oil type, are also useful in the compositions and methods describe herein. In general, such single or multiphase emulsions contain water, emollients, and emulsifiers as essential ingredients.

The compositions described herein can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

One or more additional agents can be added in the topical formulations in order to enhance the percutaneous absorption of the active ingredients, including, but not limited to, dimethylsulfoxide, dimethylacetamide, dimethylformamide, surfactants, azone (laurocapram), alcohol, acetone, propylene glycol and polyethylene glycol. Physical methods can also be used to enhance transdermal penetration such as iontophoresis or sonophoresis.

A topically applied composition provided herein contains a cosmetically effective agent that has the desired effect on skin as described herein, and those ingredients as are necessary for use as a carrier, such as an emulsion, a cream, an ointment, an aqueous solution, a lotion or an aerosol.

The carrier utilized in the compositions described herein can be in a wide variety of forms. These include emulsion carriers, including, but not limited to, oil-in-water, water-in-oil, water-in-oil-in-water, and oil-in-water-in-silicone emulsions, a cream, an ointment, an aqueous solution, a lotion or an aerosol. A given component will distribute primarily into either the water or oil/silicone phase, depending on the water solubility/dispersibility of the component in the composition.

Dermatological formulations provided herein may typically comprise a derivative of any compound or composition described herein and optionally, a polar solvent. Solvents suitable for use in the formulations described herein include any polar solvent capable of dissolving the derivative. Suitable polar solvents may include: water; alcohols (such as ethanol, propyl alcohol, isopropyl alcohol, hexanol, and benzyl alcohol); polyols (such as propylene glycol, polypropylene glycol, butylene glycol, hexylene glycol, maltitol, sorbitol, and glycerine); and panthenol dissolved in glycerine, flavor oils and mixtures thereof. Mixtures of these solvents can also be used. Exemplary polar solvents may be polyhydric alcohols and water. Examples of solvents may include glycerine, panthenol in glycerine, glycols such as propylene glycol and butylene glycol, polyethylene glycols, water and mixtures thereof. Additional polar solvents for use may be alcohols, glycerine, panthenol, propylene glycol, butylene glycol, hexylene glycol and mixtures thereof.

An emollient may also be added to the cosmetic/dermatological compositions described herein. The emollient component can comprise fats, oils, fatty alcohols, fatty acids and esters which aid application and adhesion, yield gloss and provide occlusive moisturization. Suitable emollients for use may be isostearic acid derivatives, isopropyl palmitate, lanolin oil, diisopropyl dimerate, maleated soybean oil, octyl palmitate, isopropyl isostearate, cetyl lactate, cetyl ricinoleate, tocopheryl acetate, acetylated lanolin alcohol, cetyl acetate, phenyl trimethicone, glyceryl oleate, tocopheryl linoleate, wheat germ glycerides, arachidyl propionate, myristyl lactate, decyl oleate, propylene glycol ricinoleate, isopropyl linoleate, pentaerythrityl tetrastearate, neopentylglycol dicaprylate/dicaprate, hydrogenated coco-glycerides, isononyl isononanoate, isotridecyl isononanoate, myristyl myristate, triisocetyl citrate, cetyl alcohol, octyl dodecanol, oleyl alcohol, panthenol, lanolin alcohol, linoleic acid, linolenic acid, sucrose esters of fatty acids, octyl hydroxystearate and mixtures thereof. Suitable emollients may include polar emollient emulsifiers (such as linear or branched chained polyglycerol esters) and non-polar emollients. The emollient component typically may comprise from about 1% to about 90%, preferably from about 10% to about 80%, more preferably from about 20% to about 70%, and most preferably from about 40% to about 60%, of the cosmetic composition.

By "polar emollient," as used herein, is meant any emollient emulsifier having at least one polar moiety and wherein the solubility (at 30 °C) of the cytoprotective derivative compound in the polar emollient is greater than about 1.5%, preferably greater than about 2%, more preferably greater than about 3%. Suitable polar emollients may include, but are not limited to, polyol ester and polyol ethers such as linear or branched chained polyglycerol esters and polyglycerol ethers. Non-limiting examples of such emollients may include PG3 diisostearate, polyglyceryl-2-sesquiisostearate, polyglyceryl-5-distearate, polyglyceryl-10-distearate, polyglyceryl-10-diisostearate, acetylated monoglycerides, glycerol esters, glycerol tricaprylate/caprate, glyceryl ricinoleate, glyceryl isostearate, glyceryl myristate, glyceryl linoleate, polyalkylene glycols such as PEG 600, monoglycerides, 2-monolaurin, sorbitan esters and mixtures thereof.

By "non-polar emollient," as used herein, means any emollient emulsifier possessing no or minimal permanent electric moments. Suitable non-polar emollients may include, but are not limited to, esters and linear or branched chained hydrocarbons. Non-limiting examples of such emollients may include isononyl isononanoate, isopropyl isostearate, octyl hydroxystearate, diisopropyl dimerate, lanolin oil, octyl palmitate, isopropyl palmitate, paraffins, isoparaffins, acetylated lanolin, sucrose fatty acid esters, isopropyl myristate, isopropyl stearate, mineral oil, silicone oils, dimethicone, allantoin, isohexadecane, isododecane, petrolatum, and mixtures thereof. The solubility of the compound in polar or non-polar emollients may be determined according to methods known in the art.

Suitable oils include esters, triglycerides, hydrocarbons and silicones. These can be a single material or a mixture of one or more materials. They may normally comprise from 0.1% to about 100%, preferably from about 5% to about 90%, and most preferably from about 70% to about 90% of the emollient component.

Suitable oils for use herein may be acetylglycerides, octanoates, and decanoates of alcohols and polyalcohols, such as those of glycol and glycerol, the ricinoleates of alcohols and polyalcohols such as cetyl ricinoleate, PG-3 diisostearate, polyglycerol ethers, polyglyerol esters, caprylic triglycerides, capric triglycerides, isostearic triglyceride, adipic triglyceride, phenyl trimethicone, lanolin oil, polybutene, isopropyl palmitate, isopropyl isostearate, cetyl ricinoleate, octyl dodecanol, oleyl alcohol, hydrogenated vegetable oils, castor oil, modified lanolins, octyl palmitate, lanolin oil, maleated soybean oil, cetyl ricinoleate, glyceryl trioctanoate, diisopropyl dimerate, synthetic lanolin derivatives and branched chain alcohols, sucrose esters of fatty acids, octyl hydroxystearate and mixtures thereof.

Preferably, the oils used may be selected such that the majority (at least about 75%, preferably at least about 80% and most preferably at least about 99%) of the types of oils used have solubility parameters that do not differ by more than from about 1 to about 0.1, preferably from about 0.8 to about 0.1.

A surfactant may also be added to compositions described herein, in order to confer beneficial cosmetic or application properties. Surfactants suitable for use may be those which can form emulsions and/or association structures. Surfactant emulsifier can be from 0% to about 20% of the formulation, preferably from 0% to about 15% and most preferably from about 1% to about 10%.

Examples of surface active agents which may be used in the compositions described herein include sodium alkyl sulfates, e.g., sodium lauryl sulfate and sodium myristyl sulfate, sodium N-acyl sarcosinates, e.g., sodium N-lauroyl sarcosinate and sodium N-myristoyl sarcosinate, sodium dodecylbenzenesulfonate, sodium hydrogenated coconut fatty acid monoglyceride sulfate, sodium lauryl sulfoacetate and N-acyl glutamates, e.g., N-palmitoyl glutamate, N-methylacyltaurin sodium salt, N-methylacylalanine sodium salt, sodium alpha-olefin sulfonate and sodium dioctylsulfosuccinate; N-alkylaminoglycerols, e.g., N-lauryl-diamino-ethylglycerol and N-myristyldiaminoethylglycerol, N-alkyl-N-carboxymethylammonium betaine and sodium 2-alkyl-1-hydroxyethylimidazoline betaine; polyoxyethylenealkyl ether, polyoxyethylenealkylaryl ether, polyoxyethylenelanolin alcohol, polyoxyethyleneglyceryl monoaliphatic acid ester, polyoxyethylenesorbitol aliphatic acid ester, polyoxyethylene aliphatic acid ester, higher aliphatic acid glycerol ester, sorbitan aliphatic acid ester, Pluronic type surface active agent, and polyoxyethylenesorbitan aliphatic acid esters such as polyoxyethylenesorbitan monooleate and polyoxyethylenesorbitan monolaurate. Emulsifier-type surfactants known to those of skill in the art can be used in the compositions described herein.

Also useful herein may be surfactants that form association structures, preferably lamellar or hexagonal liquid crystals, at ambient temperature when mixed with a polar solvent. In preparing a sample combination of surfactant and polar solvent to demonstrate the ability to form association structures, the surfactant needs to be sufficiently soluble in the polar solvent such that an association structure can form at ambient temperature.

Any surfactant which forms association structures at ambient temperature and is suitable for use in cosmetics may be suitable for use herein. Surfactants suitable for use in cosmetics present no or minimal dermatological or toxicological problems. Anionic surfactants, nonionic surfactants, cationic surfactants, amphoteric surfactants and mixtures thereof may be suitable for use.

The surfactants can be used at levels from about 4% to about 97%, preferably from about 5% to about 95%, more preferably from about 20% to about 90% and most preferably from about 30% to about 70% of the association structure.

The cosmetic compositions described herein may contain one or more materials, herein singly or collectively referred to as a "solidifying agent", that are effective to solidify the particular liquid base materials to be used in a cosmetic composition. (As used herein, the term "solidify" refers to the physical and/or chemical alteration of the liquid base material so as to form a solid or semi-solid at ambient conditions, i.e., to form a final composition that has a stable physical structure and can be deposited on the skin under normal use conditions.) As is appreciated by those skilled in the art, the selection of the particular solidifying agent for use in the cosmetic compositions will depend upon the particular type of composition desired, i.e., gel or wax-based, the desired rheology, the liquid base material used and the other materials to be used in the composition. The solidifying agent can be preferably present at a concentration of from about 0.1% to about 90%, more preferably from about 1% to about 50%, even more preferably from about 5% to about 40%, most preferably from about 3% to about 20%.

The wax cosmetic stick variations provided herein preferably may contain from about 5% to about 50% (by weight) of a waxy solidifying agent. By the term "waxy solidifying agent," as used herein, is meant a solidifying material having wax-like characteristics. Such waxy materials may also serve as emollients. Among the waxy materials useful herein are the high melting point waxes, i.e., having a melting point of from about 65°C to about 125°C, such as beeswax, spermaceti, carnauba, bayberry, candelilla, montan, ozokerite, ceresin, paraffin, synthetic waxes such as Fisher-Tropsch waxes, microcrystalline wax, and mixtures thereof. Ceresin, ozokerite, white beeswax, synthetic waxes, and mixtures thereof, are among those useful herein. Low melting waxes, having a melting point of from about 37° C to about 75° C, may be preferred for use in the wax stick variations described herein. Wax stick variations, which contain volatile silicone oils as a liquid base material, preferably contain from about 10% to about 35%, more preferably from about 10% to about 20% (by weight), of a low-melting wax. Such materials include fatty acids, fatty alcohols, fatty acid esters and fatty acid amides, having fatty chains of from about 8 to about 30 carbon atoms, and mixtures thereof. Wax-like materials include cetyl alcohol, palmitic acid, stearyl alcohol, behenamide, sucrose esters of tallow fatty acids, mono and di-fatty acid esters of polyethylene glycol, and mixtures thereof. Stearyl alcohol, cetyl alcohol, and mixtures thereof, are mostly used. Additional fatty acids, fatty alcohols, and other wax-like materials are also well known in the art.

In addition, these compositions may include other cosmetic agents, carriers, adjuvants, and the like. Some particular additional agents may include sunscreens; retinoids; antioxidants; hydroxyacids; fatty acids, acceptable non-toxic metal salts of naturally occurring amino acids or of hydroxyalkyl acids; botanical extracts, salicylic acid, benzoyl peroxide, antibiotics, antiandrogens, anti-inflammatory agents, antioxidants, ascorbic acid, vitamins B, tocopherols or tocotrienols, corticosteroids, moisteners, surfactants, keratolytic agents, complexing agents, colorants, fragrances, and mixtures thereof.

### Synthetic methods

The resorcinol compounds described herein can be synthesized by an appropriate combination of generally well-known synthetic methods. Techniques useful in synthesizing the compounds herein are both readily apparent and accessible to those of skill in the relevant art in light of the teachings described herein. 4-alkyl resorcinols are commercially available from Sigma-Aldrich, St. Louis, Missouri, USA.

### Examples

### EXAMPLE 1

### Evaluation for Signs of Degradation and Stabilization of 4-Alkyl Resorcinols

### Color Measurements

Samples for color evaluation were prepared by dissolving 4-hexylresorcinol (200mg) or 4-ethylresorcinol (200mg) in DI water:butylene glycol (4:1; 20ml) to generate a clear colorless homogeneous solution. A portion of each solution (5ml) was added to a 20ml scintilation vial containing either niacinamide (B3) (50mg), AceMet (acetyl methionine) (50mg) or B3 (50mg) + AceMet (50mg) and monitored @ R.T. (~20-22 °C). After 2 weeks, the color of each solution was assessed visually and L*a*b* measurements determined experimentally using a Labscan XE instrument (Hunter Associates Labs Inc., Reston, VA) and processed with Universal Software (version 4.10). The L* parameter measures darkness and lightness and ranges from black (L* = 0) to white (L* = 100). The a* parameter measures color content and intensity ranging from green (a* < 0) to neutral grey (a* = 0) to red (a* > 0). The b* parameter also measures color content and intensity ranging from blue (b* < 0) to neutral grey (b* = 0) to yellow (b* > 0).

### Quantitation of 4-Alkyl Resorcinols

The amount of 4-alkylresorcinol present in each of the samples used for the color measurements was quantitated after 2 weeks @ R.T. (-20-22 °C) by high performance liquid chromatography (HPLC). An sample aliquot (100uL) from each of the color measurement solutions was diluted with DI water: acetonitrile (1:1, 900uL) to generate test samples (1ml). An aliquot (10uL) from each test sample was injected into a Waters 2695 Separations Module, chromatographed and separated on a Restek Pinnacle DB C18 (5um, 4.6 × 150mm) column operated at 30 °C and the 4-alkylresorcinols monitored using a Waters 2996 Photodiode Array Detector set @ 281nm. The mobile phase consisted of water (A) and acetonitrile (B) and optimum separation was achieved using a gradient from 95:5 (A:B) to 5:95 (A:B) in 20min, followed by isocratic elution at 5:95 (A:B) for 3min at a flow rate of 1 ml/min. The chromatographic data was processed using Empower 2 software (Waters Corporation, Milford, MA). Standard control samples of 4-alkylresorcinols at 1X, 5X higher and 5X lower concentrations were prepared fresh and immediately analysed; standard curves were generated within the test sample range and linearity was confirmed.

All samples were analyzed in duplicate and the mean value reported. The amount of 4-alkylresorcinol present for each test sample after 2 weeks was determined by measuring the chromatographic peak area under the curve @ 281 nm for the 4-alkylresorcinol and expressed as the percent ratio over the corresponding chromatographic peak area under the curve @ 281 nm for the freshly prepared control sample.

Table 1 below shows the effect of AceMet on the color and stability of 4-alkylresorcinols (hexyl resorcinol "HR" and ethyl resorcinol "ER") in the presence and absence of niacinamide "B3." A freshly prepared solution of HR is colorless and no decomposition of HR is observed upon HPLC analysis. After 2 weeks, the color of the HR solution changes to a reddish-pink color as demonstrated by the increases in a* and b* components (as well as a decrease in L*) and the level of HR decreases by 40%. Addition of B3 to the HR solution significantly accelerates decomposition of HR by 55% and generates a more intense color as seen by further significant increases in a*, b* and decreases in L*. In contrast and unexpectedly, addition of AceMet to the HR solution significantly prevents color formation and HR decomposition in the presence or absence of B3 as demonstrated by the corresponding L*a*b* values and HR content which are very close to control levels Similarly, AceMet significantly prevented color formation in ER solutions in the presence or absence of B3 and offered protection against ER decomposition, albeit to a lesser degree compared to HR. These results clearly demonstrate the significant benefits of AceMet on color and chemical stabilization of 4-alkylresorcinols in the presence or absence of other cosmetic ingredients (such as B3) that can further accelerate such color formation and decomposition.

The effect of AceMet on color and stability of 4-Alkylresorcinols (HR and ER) in the presence and absence of B3 after 2 weeks at R.T. (~20-22 °C) is shown in the Table below:

**TABLE 1**

| Examples | Sample ^{a} | Appearance ^{b} | L* | a* | b* | 4-Alkylresorcinol (% of control) ^{c} |
|---|---|---|---|---|---|---|
| 1 | Control | colorless | 98 | 0 | 0 | 100 |
| 2 | 1% HR | reddish-pink | 84.77 | 11.4 | 13.4 | 60 |
| 3 | 1% HR + 1% B3 | orange | 80.87 | 17.97 | 36.52 | 45 |
| 4 | 1% HR + 1% AceMet | nearly colorless | 94.61 | -0.85 | 2.32 | 107 |
| 5 | 1% HR + 1% B3 + 1% AceMet | nearly colorless | 94.61 | -0.85 | 2.32 | 106 |
| 6 | 1% ER | light orange | 82.74 | 6.38 | 39.57 | 96 |
| 7 | 1% ER + 1% B3 | yellow-orange | 78.81 | 7.33 | 39.31 | 90 |
| 8 | 1% ER + 1% AceMet | nearly colorless | 94.61 | -0.85 | 2.32 | 102 |
| 9 | 1% ER + 1% B3 + 1% AceMet | nearly colorless | 94.61 | -0.85 | 2.32 | 102 |

| | | | | | | |
|---|---|---|---|---|---|---|
| ^{a} Control = DI water:butylene glycol (4:1). ^{b} Visual appearance of solution after 2 weeks. ^{c} Determined by HPLC analysis. | | | | | | |

### EXAMPLE 2. ER and AceMet

A topical cosmetic composition within the scope of the invention was prepared. A base formulation shown in the Table below was made by heating phase A ingredients to 70 to 85° C with stirring. Phase B ingredients were heated in a separate container to 70 to 85° C with stirring. Then, phase A was added into phase B while both phases were kept at 70 to 85° C. The mixture was stirred for at least 15 minutes at 70 to 85° C, then cooled. Phase C ingredients were added at 50° C, followed by Phase D ingredients added at 40 ° C.

**TABLE 2. Base Formulation**

| | a | b | |
|---|---|---|---|
| Ingredients | %wt. | %wt. | Phase |
| Isostearyl Palmitate | 6.00 | 6.00 | A |
| C12-C15 Alkyl Octanoate | 3.00 | 3.00 | A |
| PEG-100 Stearate | 2.00 | 2.00 | A |
| Glyceryl Hydroxystearate | 1.50 | 1.50 | A |
| Stearyl Alcohol | 1.50 | 1.50 | A |
| Stearic acid | 3.00 | 4.00 | A |
| TEA, 99% | 1.20 | 1.20 | B |
| Dimethicone | 1.00 | 1.00 | A |
| Sorbitan Monostearate | 1.00 | 1.00 | A |
| Magnesium Aluminum Silicate | 0.60 | 0.60 | B |
| Vitamin E acetate | 0.10 | 0.10 | A |
| Cholesterol | 0.50 | 0.50 | A |
| Simethicone | 0.01 | 0.01 | B |
| Xanthan gum | 0.20 | 0.20 | B |
| Hydroxyethylcellulose | 0.50 | 0.50 | B |
| Propylparaben | 0.10 | 0.10 | A |
| Disodium EDTA | 0.05 | 0.05 | B |
| Fragrance components | 0.50 | 0.50 | D |
| Niacinamide | 0.05 | 0.05 | C |
| BHT | 0.10 | 0.10 | C |
| 4-ethyl resorcinol | 0.05 | 2.00 | C |
| AceMet | 0.0005 | 0.001 | D |
| Methylparaben | 0.15 | 0.15 | B |
| Water | BAL | BAL | B |
| Total | 100.00 | 100.00 | |

### EXAMPLE 3

An additional cosmetic composition was prepared as shown in the Table below The composition comprises 4-cyclohexyl resorcinol which is not part of the invention as claimed.

**TABLE 3**

| | Wt% | Phase |
|---|---|---|
| water, DI | BAL | A |
| disodium EDTA | 0.05 | A |
| magnesium aluminum silicate | 0.6 | A |
| methyl paraben | 0.15 | A |
| simethicone | 0.01 | A |
| butylene glycol 1,3 | 3.0 | A |
| hydroxyethylcellulose | 0.5 | A |
| glycerine, USP | 2.0 | A |
| xanthan gum | 0.2 | A |
| triethanolamine | 1.2 | B |
| stearic acid | 3.0 | B |
| propyl paraben NF | 0.1 | B |
| glyceryl hydroxystearate | 1.5 | B |
| stearyl alcohol | 1.5 | B |
| isostearyl palmitate | 6.0 | B |
| C12-15 alcohols octanoate | 3.0 | B |
| dimethicone | 1.0 | B |
| cholesterol NF | 0.5 | B |
| sorbitan stearate | 1.0 | B |
| Fragrance components | 1.0 | B |
| tocopheryl acetate | 0.1 | B |
| PEG-100 stearate | 2.0 | B |
| sodium stearoyl lactylate | 0.5 | B |
| hydroxycaprylic acid | 0.1 | C |
| 4-cyclohexyl Resorcinol | 10.0 | C |
| AceMet | 0.001 | D |
| BHT | 0.10 | C |
| Niacinamide | 0.05 | C |
| alpha-bisabolol | 0.2 | C |

The composition was prepared as follows:
1. Heat Phase A to 80°C while mixing.
2. Heat Phase B to 75°C in a separate container while mixing.
3. Add B to A and mix with heat maintained at 70 - 80°C for 15 min; then heat turned off and continued mixing for another 15 min.
4. At 50°C add Phase C and mix for 10 min.
5. At 40°C add Phase D and mix for 10 min.

### EXAMPLES 4 - 11

A set of additional compositions within the scope of the present invention were prepared by the method of Example 2 and are listed in the table below.

**TABLE 4. HR and AceMet**

| Ingredients | Phase | Examples (wt. %) | | | | | | | |
|---|---|---|---|---|---|---|---|---|---|
| | | 4 acid soap base | 5 | 6 | 7 | 8 | 9 | 10 | 11 |
| Stearic acid | A | 17.9 | 17.9 | 17.9 | 17.9 | 17.9 | 17.9 | 17.9 | 17.9 |
| Sodium cetearyl sulfate | A | | 2.2 | | 1 | 1.5 | 2 | 3 | 2 |
| Myrj 59 | A | | | 2 | 2 | 2 | 2 | 2 | 1 |
| Propylparaben | A | 0.1 | 0.1 | 0.1 | 0.1 | 0.1 | 0.1 | 0.1 | 0.1 |
| Disodium EDTA | A | 0.05 | 0.05 | 0.05 | 0.05 | 0.05 | 0.05 | 0.05 | 0.05 |
| Span 60 | A | | | 2 | 2 | 2 | 2 | 2 | 1 |
| BHT | C | 0.10 | 0.10 | 0.10 | 0.10 | 0.10 | 0.10 | 0.10 | 0.10 |
| 4-Hexyl resorcinol | C | 0.05 | 0.05 | 2.0 | 2.0 | 3.5 | 3.5 | 5.0 | 10.0 |
| AceMet | D | 0.001 | 0.001 | 0.01 | 0.01 | 0.5 | 0.5 | 0.001 | 0.01 |
| KOH, 22% | B | 2.20 | | | | | | | |
| Glycerin | B | 1 | 1 | 1 | 1 | 1 | 1 | 1 | 1 |
| Methylparaben | B | 0.15 | 0.15 | 0.15 | 0.15 | 0.15 | 0.15 | 0.15 | 0.15 |
| Water | B | BAL | BAL | BAL | BAL | BAL | BAL | BAL | BAL |

### EXAMPLE 12

**The formulations in the Table below were prepared in accordance with the method set forth in Example 1 hereinabove**. The formulations of examples 4 and 5 comprise 4-cyclohexyl resorcinol which is not part of the invention as claimed.

**TABLE 5: Formulations for Skin Lotions**

| | | | 1 | 2 | 3 | 4 | 5 |
|---|---|---|---|---|---|---|---|
| Raw Material | | | Wt % | Wt % | Wt % | Wt % | Wt % |
| | | | | | | | |
| Stearic acid | A | | 17.90 | 17.90 | 17.90 | 17.90 | 17.90 |
| Cetostearyl Alcohol | A | | 0.53 | 0.53 | 0.53 | 0.53 | 0.53 |
| Dimethicone (DC200, 350 cSt) | A | | 0.50 | 0.50 | 0.50 | 0.50 | 0.50 |
| Parsol MCX | A | | 1.25 | 1.25 | 1.25 | 1.25 | 1.25 |
| Parsol 1789 | A | | 0.40 | 0.40 | 0.40 | 0.40 | 0.40 |
| Propyl paraben | A | | 0.10 | 0.10 | 0.10 | 0.10 | 0.10 |
| | | | | | | | |
| | | | | | | | |
| Water | B | | 55.88 | 55.88 | 55.88 | 55.88 | 55.88 |
| Glycerin | B | | 1.00 | 1.00 | 1.00 | 1.00 | 1.00 |
| EDTA | B | | 0.04 | 0.04 | 0.04 | 0.04 | 0.04 |
| Methyl paraben | B | | 0.20 | 0.20 | 0.20 | 0.20 | 0.20 |
| KOH, 22% | B | | 2.20 | 2.20 | 2.20 | 2.20 | 2.20 |
| | | | | | | | |
| AceMet | D | | 1.0 | 1.0 | 1.0 | 1.0 | 1.0 |
| | | | | | | | |
| Niacinamide | C | | 1.00 | 1.00 | ------- | ------- | 1.00 |
| BHT | C | | 0.10 | ------- | ------- | ------- | ------- |
| 4-Ethyl Resorcinol | C | | 2.00 | 3.55 | ------- | ------- | ------- |
| 4-Hexyl Resorcinol | C | | --------- | -------- | 5.00 | ------- | -------- |
| 4-cyclohexyl Resorcinol | C | | --------- | -------- | ------- | 2.84 | 6.00 |
| Q.S. to 100% with water | | | Q.S. | Q.S. | Q.S. | Q.S. | Q.S. |

## Claims

1. A stabilized topical cosmetic composition comprising:
a. 0.00001 to 10 wt.% of said composition of N-Acetyl Methionine;
b. 0.00001 to 10 wt.% of said composition of one or more 4-substituted resorcinol derivatives of
the general formula I: 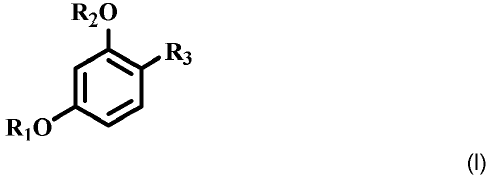
wherein each **R₁** and **R₂** independently is selected from the group consisting of a hydrogen atom, -CO-R, -COO-R, and CONHR; wherein R represents a C₁ - C₁₈ hydrocarbon; and
wherein **R₃** represents a C₁ - C₁₈ hydrocarbon or has a general formula (II): 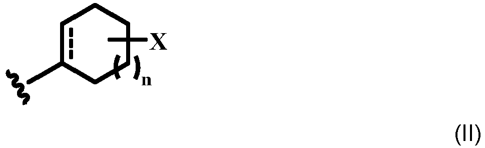
wherein X represents hydrogen; OR¹, wherein R¹ represents hydrogen, (C₁ - C₆)alkyl or aryl-(C₁-C₆)alkyl; OCOR² wherein R² represents (C₁-C₆)alkyl, aryl-(C₁-C₆)alkyl or phenyl; (C₁-C₆)alkyl; aryl-(C₁-C₆)alkyl; or NHR¹ wherein R¹ is defined as above; n is 0 to 3; and the dashed line indicates an optional double bond; and
c. a cosmetically acceptable vehicle; wherein **R₃** is ethyl.

2. The composition of claim 1, wherein both **R₁** and **R₂** are H.

3. The cosmetic composition of claim 1 or claim 2, , further comprising a skin benefit agent selected from the group consisting of alpha-hydroxy acids and esters, beta-hydroxy acids and esters, polyhydroxy acids and esters, kojic acid and esters, ferulic acid and ferulate, vanillic acid and esters, dioic acids and esters, retinol, retinal, retinyl esters, hydroquinone, t-butyl hydroquinone, 1-methylnicotinamide chloride and mixtures thereof.

4. The stabilized cosmetic composition of any one of claims 1 to 3,
wherein said N-Acetyl Methionine is present in an amount of 0.0001 wt % to 5 wt % of said cosmetic composition; and wherein the weight ratio of said N-Acetyl Methionine to said 4-substituted resorcinol derivatives of the general formula I is 10,000:1 to 1:100,000.

5. A method of stabilizing 4-ethyl resorcinol or 4-hexyl resorcinol in a composition comprising a cosmetically acceptable vehicle by adding to the composition Acetyl Methionine in an amount sufficient to stabilize said 4-ethyl resorcinol or 4-hexyl resorcinol.

6. Use of N-Acetyl Methionine for stabilizing 4-ethyl resorcinol or 4-hexyl resorcinol in a composition comprising a cosmetically acceptable vehicle.

## Patentansprüche

1. Stabilisierte topische kosmetische Zusammensetzung, umfassend:
a. 0,00001 bis 10 Gew.-% der Zusammensetzung N-Acetylmethionin;
b. 0,00001 bis 10 Gew.-% der Zusammensetzung eines oder mehrerer 4-substituierter Resorcin-Derivate der allgemeinen Formel I: 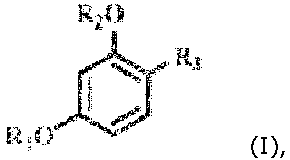
worin jedes R₁ und R₂ unabhängig aus der Gruppe ausgewählt ist, bestehend aus einem Wasserstoffatom, -CO-R, -COO-R und CONHR; wobei Reinen C₁-C₁₈-Kohlenwasserstoff darstellt; und
wobei R₃ einen C₁-C₁₈-Kohlenwasserstoff darstellt oder eine allgemeine Formel (II) 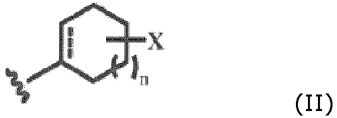 aufweist,
wobei X darstellt Wasserstoff; OR¹,
wobei R¹ darstellt Wasserstoff, (C₁-C₆)Alkyl oder Aryl-(C₁-C₆)Alkyl; OCOR², wobei R² darstellt (C₁-C₆)Alkyl, Aryl-(C₁-C₆)Alkyl oder Phenyl; (C₁-C₆)Alkyl; Aryl-(C₁-C₆)Alkyl; oder NHR¹, wobei sich R¹ wie oben definiert darstellt;
n 0 bis 3 ist und die gestrichelte Linie eine optionale Doppelbindung ist; und
c. ein kosmetisch akzeptables Vehikel, wobei R₃ Ethyl ist.

2. Zusammensetzung nach Anspruch 1, wobei sowohl R₁ als auch R₂ H sind.

3. Kosmetische Zusammensetzung nach Anspruch 1 oder Anspruch 2, weiter umfassend ein Hautpflegemittel, das aus der Gruppe ausgewählt ist, bestehend aus Alpha-Hydroxysäuren und -estern, Beta-Hydroxysäuren und -estern, Polyhydroxysäuren und -estern, Kojisäure und -estern, Ferulasäure und -Ferulat, Vanillinsäure und -estern, Disäuren und -estern, Retinol, Retinal, Retinylestern, Hydrochinon, t-Butylhydrochinon, 1-Methylnikotinamidchlorid und Mischungen davon.

4. Stabilisierte kosmetische Zusammensetzung nach irgendeinem der Ansprüche 1 bis 3, wobei das N-Acetylmethionin in einer Menge von 0,0001 Gew.-% bis 5 Gew.-% der kosmetischen Zusammensetzung vorliegt; und wobei das Gewichtsverhältnis des N-Acetylmethionins zu den 4-substituierten Resorcin-Derivaten der allgemeinen Formel I 10.000:1 bis 1:100.000 beträgt.

5. Verfahren zur Stabilisierung von 4-Ethylresorcin oder 4-Hexylresorcin in einer Zusammensetzung, umfassend ein kosmetisch akzeptables Vehikel durch Zugabe von Acetylmethionin zu der Zusammensetzung in einer Menge, die ausreicht, das 4-Ethylresorcin oder 4-Hexylresorcin zu stabilisieren.

6. Verwendung eines N-Acetylmethionins zum Stabilisieren von 4-Ethylresorcin oder 4-Hexylresorcin in einer Zusammensetzung, umfassend ein kosmetisch akzeptables Vehikel.

## Revendications

1. Composition cosmétique stabilisée à usage topique comprenant :
a. 0,00001 à 10 %, en poids de ladite composition, de N-acétylméthionine ;
b. 0,00001 à 10 %, en poids de ladite composition, d'un ou plusieurs dérivés de résorcinol 4-substitués de formule générale I : 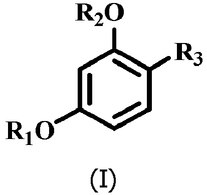
dans laquelle chaque R₁ et R₂ est indépendamment choisi dans le groupe constitué par un atome d'hydrogène, -CO-R, -COO-R, et CONHR ; où R représente un hydrocarbure en C₁ à C₁₈ ; et
dans laquelle R₃ représente un hydrocarbure en C₁ à C₁₈ ou répond à la formule générale (II) : 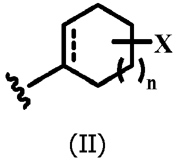
dans laquelle X représente ; l'hydrogène ; OR¹, où R¹ représente l'hydrogène, un alkyle en C₁ à C₆ ou un aryl-alkyle en C₁ à C₆ ; OCOR² où R² représente un alkyle en C₁ à C₆, un aryl-alkyle en C₁ à C₆ ou phényle ; un alkyle en C₁ à C₆ ; un aryl-alkyle en C₁ à C₆ ; ou NHR¹ où R¹ est tel que défini ci-dessus ; n vaut de 0 à 3 ; et la ligne de tirets indique une double liaison optionnelle ; et
c. un véhicule acceptable en cosmétique ;
dans laquelle R₃ est éthyle.

2. Composition selon la revendication 1, dans laquelle R₁ et R₂ sont tous deux H.

3. Composition cosmétique selon la revendication 1 ou la revendication 2, comprenant en outre un agent bénéfique pour la peau choisi dans le groupe constitué par les alpha-hydroxyacides et leurs esters, les bêta-hydroxyacides et leurs esters, les polyhydroxyacides et leurs esters, l'acide kojique et ses esters, l'acide férulique et un férulate, l'acide vanillique et ses esters, les acides dioïques et leurs esters, le rétinol, le rétinal, les esters rétinyliques, l'hydroquinone, la t-butylhydroquinone, le chlorure de 1-méthylnicotinamide et leurs mélanges.

4. Composition cosmétique stabilisée selon l'une quelconque des revendications 1 à 3, dans laquelle ladite N-acétylméthionine est présente en une quantité de 0,0001 % en poids à 5 % en poids de ladite composition cosmétique ; et dans laquelle le rapport en poids de ladite N-acétylméthionine auxdits dérivés de résorcinol 4-substitués de formule générale (I) est de 10 000/1 à 1/100 000.

5. Procédé de stabilisation de 4-éthylrésorcinol ou de 4-hexylrésorcinol dans une composition comprenant un véhicule acceptable en cosmétique par addition à la composition d'acétylméthionine en une quantité suffisante pour stabiliser ledit 4-éthylrésorcinol ou 4-hexylrésorcinol.

6. Utilisation de N-acétylméthionine pour stabiliser le 4-éthylrésorcinol ou le 4-hexylrésorcinol dans une composition comprenant un véhicule acceptable en cosmétique.
